# FASCICULE DE BREVET EUROPEEN

(11) **EP 2 257 523 B1**
(45) Date de publication et mention de la délivrance du brevet: **10.08.2011**
(21) Numéro de dépôt: 09722166.7
(22) Date de dépôt: 06.03.2009
(51) Int. Cl.: C07C 235/34

(54) **PRÉPARATION DE COMPOSÉS HYDROQUINONE AMIDE À PROPRIÉTÉS ANTIOXYDANTES**
ZUBEREITUNG VON HYDROCHINONAMIDVERBINDUNGEN MIT ANTIOXIDATIONSEIGENSCHAFTEN
PREPARATION OF HYDROQUINONE AMIDE COMPOUNDS WITH ANTIOXIDANT PROPERTIES

(30) Priorité: 07.03.2008 FR 0801246
(43) Date de publication de la demande: 08.12.2010
(73) Titulaire: Catalys Sas, 69446 Lyon Cedex 03 (FR)
(72) Inventeur: ADRIAN, Guy, F-69230 Saint-Genis-Laval (FR); BIGOT, Patrick, F-69008 Lyon (FR)
(74) Mandataire: Maureau, Philippe
(86) Numéro de dépôt international: PCT/FR2009/000233
(87) Numéro de publication internationale: WO 2009/115707

(56) Documents cités:
- EP-A- 0 900 781
- WO-A-01/98258
- WO-A-2006/111233
- YABUNAKA H ET AL: "Hybrid ubiquinone: novel inhibitor of mitochondrial complex I" BIOCHIMICA ET BIOPHYSICA ACTA. BIOENERGETICS, AMSTERDAM, NL, vol. 1556, no. 2-3, 2 décembre 2002 (2002-12-02), pages 106-112, XP004396758 ISSN: 0005-2728

## Description

La présente invention concerne la préparation de composés hydroquinones-amides à propriétés antioxydantes dérivés d'un acide arylacétique comportant au moins deux groupes hydroxy et amines ou amino-alcanols correspondants à la formule (I) 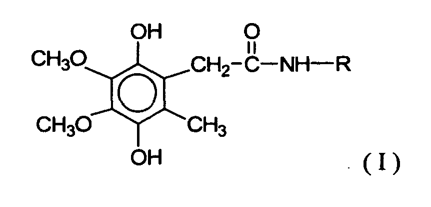
avec R = alkyl (-CₙH₂ₙ₊₁)
ou R = hydroxyalkyl (-CₙH₂ₙ₊₁O)
ou R = dihydroxyalkyl (-CₙH₂ₙ₊₁O₂)
n étant compris entre 1 et 30.

Les composés (I) possèdent la structure aromatique des coenzymes Q réduits ou ubiquinols de formule (II) 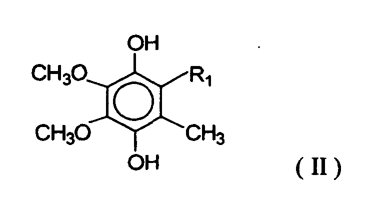 eux-mêmes forme réduite des coenzymes Q ou ubiquinones de formule (III) connues pour leurs propriétés antioxydantes et inhibitrices de radicaux libres chez l'homme (B. Halliwell et J. Gutteridge - Free radicals in Biology and Medicine - 1998).

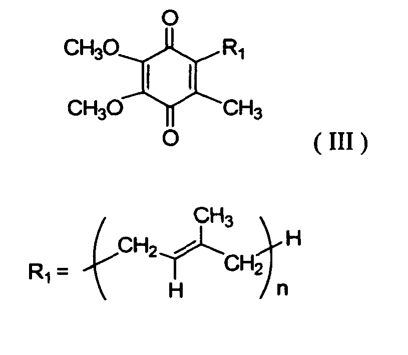

Les ubiquinones ou coenzymes Qₙ où n est le nombre d'unités isoprène dans la chaîne R₁, sont des molécules lipophiles constituées d'une partie benzoquinone et d'une chaîne polyisoprène-trans hydrophobe qui assure leur stabilité en milieu organique.

Chez l'homme en particulier la coenzyme Q₁₀ de formule (III) avec n = 10, après réduction « in-vivo » en hydroquinone de formule (II) selon la réaction réversible (1), est impliquée dans les mitochondries lors de la respiration cellulaire, dans la production d'adénosine triphosphate (ATP) et dans le ralentissement du vieillissement cellulaire en captant les radicaux libres selon la réaction (2).
(S. Yamashita - Detection of ubiquinol and ubiquinone as a marker of oxidative stress, Anal. Biochemistry, 1997, 250 p. 66).

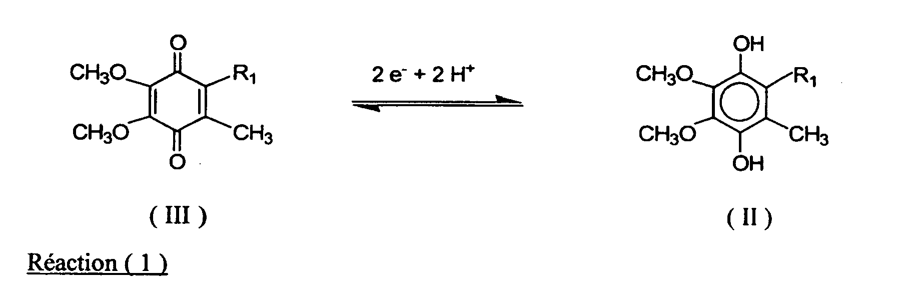

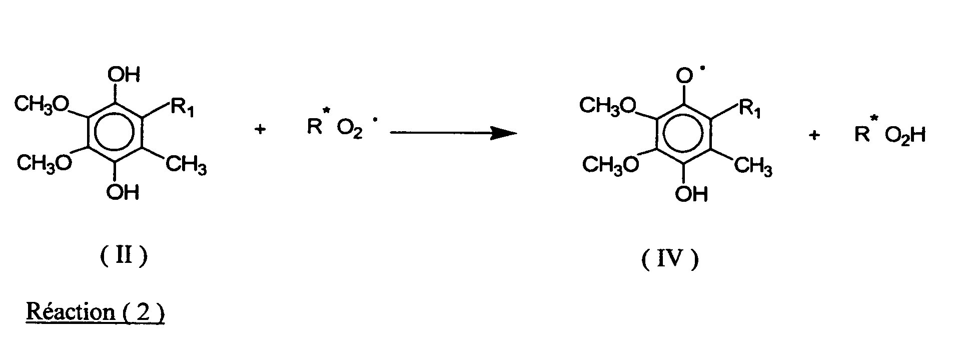

Les composés (III) sont obtenus industriellement par des procédés de fermentation ou de synthèse multi-étapes coûteux qui limitent leur utilisation à grande échelle comme antioxydant pour la prévention des maladies dégénératrices et du vieillissement humain : aspect de la peau (rides), maladies cardio-vasculaires, maladies neuro-dégénératrices et cancers, en captant efficacement les radicaux libres qui dégradent les membranes cellulaires et les lipoprotéines.

Le but de l'invention est d'obtenir des composés antioxydants dotés des propriétés ci-dessus, stables et non-toxiques, miscibles dans les milieux lipidiques et dans les milieux aqueux, permettant une pénétration efficace au niveau des tissus cutanés.

La demande de brevet WO 01/98258 divulgue des composés de type alkylamide d'acide 3,4-didydroxymandelique qui sont utilisés comme antioxydants dans des compositions pharmaceutiques et cosmétiques.

Ces composés (I) présentent le noyau aromatique propre aux ubiquinols (II) et se prêtent à la réaction d'oxydo-réduction (1).

Ils sont obtenus par amidation de l'acide dihydroxy-2,5 diméthoxy-3,4 methyl-5 benzèneacétique (V) sous forme de son dérivé cyclique (VI), par condensation avec une amine de formule RNH₂ où R représente :
- soit une chaîne R' alkyl linéaire ou branchée comportant 1 à 30 atomes de carbone : formule (Ia).
- soit une chaîne hydroxy alkyl : 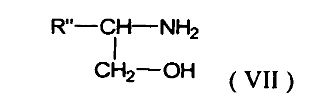 où R" est un atome d'hydrogène ou une chaîne alkyl linéaire ou branchée de 1 à 28 atomes de carbone. Pour les usages pré-cités on préfère l'amino-2-dodécanol-1, avec R"= decyl (n-C₁₀H₂₁) : formule (Ib).
- soit une chaîne dihydroxy alkyl : 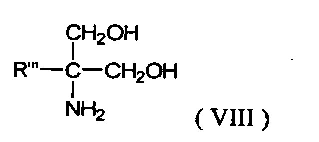 où R"' est un atome d'hydrogène ou une chaîne alkyl linéaire ou branchée de 1 à 27 atomes de carbone. Pour les usages pré-cités on préfère l'amino-2 propane diol-1,3, avec R"'= H : formule (Ic).

La formation des amides (I) à partir des amines R'NH₂ ou des aminoalcanols (VII) ou (VIII), est effectuée par simple chauffage en l'absence d'air d'un équivalent molaire de la lactone phénol (VI) en milieu solvant : réaction (3).

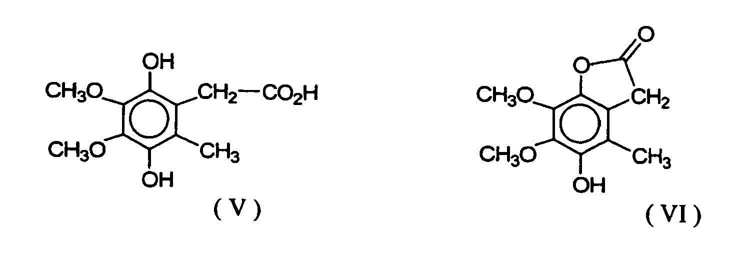

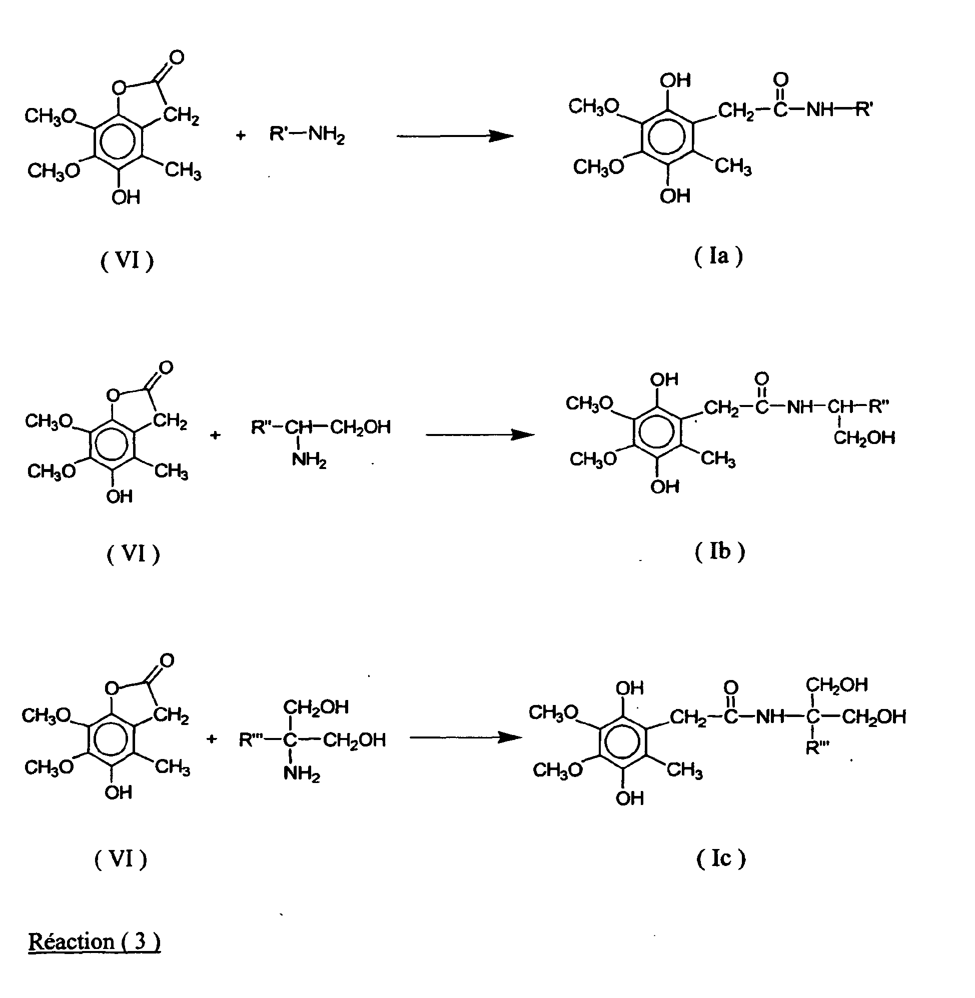

La lactonephénol (VI) n'a été décrite qu'une fois (V.G. Ramsey, Journal of the American Chemical Society 1966, p. 1553).

Elle est obtenue par ozonolyse de la coenzyme Q₉ (formule (III) avec n = 9), réduction par le zinc, acétylation puis hydrolyse en milieu acide.

Le coût élevé de la matière première, ainsi que le danger lié à l'utilisation industrielle de l'ozone, nous ont conduit à développer une méthode nouvelle de synthèse du composé (VI), en 3 étapes, à partir de la coenzyme Q₀ (formule (III) avec n = 0), matière première industrielle de la coenzyme Q₁₀, par réduction en hydroquinone de formule (II) où R₁= H.

Cette réduction est effectuée par le dithionite de sodium en milieu aqueux avec un rendement quantitatif.

La condensation avec le glyoxylate d'éthyle en présence d'un acide de Lewis, notamment le tetrachlorure de titane entre -10°C et 60°C et en particulier à 0°C, permet d'obtenir le composé nouveau (IX) avec un rendement quantitatif. (Synthesis 2004, p. 760).

L'hydrogénation de (IX) en présence de palladium sur charbon en milieu acide tel que l'acide acétique en présence d'un acide fort et en particulier en présence d'acide sulfurique entre 0°C et 120°C, de préférence à 90°C, conduit directement à la lactone (VI) avec un rendement isolé de 68 % à partir de la coenzyme Q₀ : réaction (4):

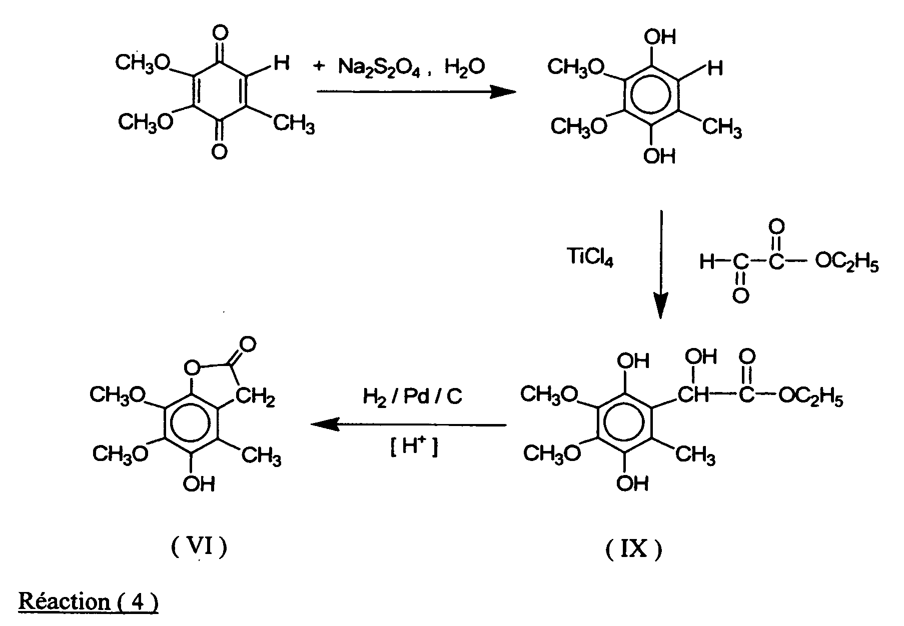

La lactone (VI), composé solide, stable, est de façon inattendue et avantageuse, directement condensée avec les amines RNH₂, sans activation ni protection de la fonction phénol libre, pour conduire directement aux composés (I).

La réaction des amines ou des aminoalcanols avec (VI) est effectuée par simple chauffage entre 50°C et 150°C dans un solvant polaire aprotique tel que la N-méthylpyrrolidone, le diméthylformamide, ou la pyridine, en l'absence d'oxygène et de préférence en présence d'un agent réducteur tel qu'un sulfite alcalin et de préférence le dithionite de sodium.

Après hydrolyse, les hydroquinones-amides (I) sont isolées selon les techniques appropriées et purifiées par cristallisation.

Les composés (I) stables à l'état solide s'oxydent en présence d'oxygène en solution ou en présence d'agents oxydants, pour conduire aux quinones-amides (X).

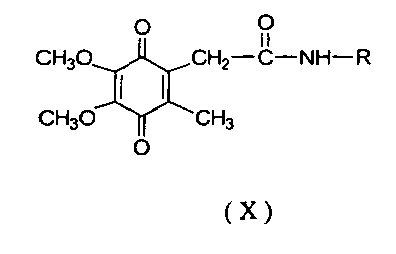

L'agent oxydant préféré est le chlorure ferrique en milieu alcool-eau, entre 0°C et 80°C, de préférence en milieu ethanol-eau à 20°C.

Les quinone-amides solides jaunes sont purifiées par cristallisation.

Le potentiel antioxydant en solution des composés (I) est évalué par mesure de leur réaction avec le radical libre diphényl-picrylhydrazide (XI) selon C.T. Ho, S. Agric. Food Chem. 1999, p. 3975, en prenant comme référence le gallate de méthyle (XII).

Les composés (I) dans leur ensemble présentent une activité antioxydante proche de celle de référence.

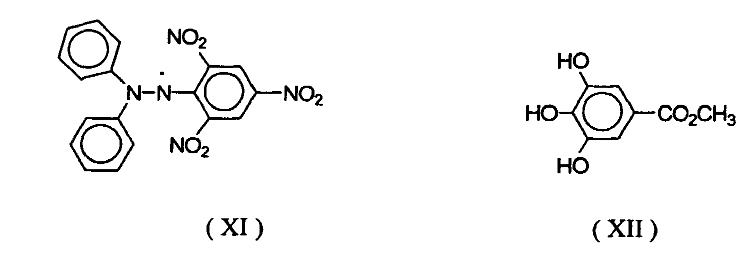

La demanderesse a mis au point un procédé de préparation de composés hydroquinone-amides à propriétés antioxydantes.

Ces composés peuvent être façonnés en comprimés, capsules, ou en mélange dans les préparations cosmétiques ou pharmaceutiques selon les techniques habituelles.

Bien entendu, diverses modifications peuvent être apportées par l'homme de l'art aux dispositifs ou procédés qui viennent d'être décrits uniquement à titre d'exemple non limitatif, sans sortir du cadre de l'invention.

L'invention est illustrée par les exemples 1 à 5 suivants :

### Exemple 1

### Obtention de la lactone de formule (VI) à partir de diméthoxy-2,3 methyl-5 benzoquinone-1,4 (coenzyme Q₀) :

On prépare une solution de 9,2 g (0,0505 mole) de diméthoxy-2,3 methyl-5 benzoquinone-1,4 dans 100 ml d'acétate d'éthyle à 20°C.
On ajoute une solution de 31 g (0,178 mole) de dithionite de sodium dans 150 ml d'eau et on agite 30 minutes.
Le milieu rouge se décolore en jaune pâle.
La phase organique est séparée et concentrée sous vide pour obtenir 9,2 g de solide beige.
F = 77°C.
Ce solide est dissout dans 70 ml de dichlorométhane, refroidi à 0°C.
Puis une solution commerciale de 10,7 g (0,0525 mole) de glyoxylate d'éthyle à 50 % dans le toluène est ajoutée, suivie de 5,5 ml (0,05 mole) de chlorure de titane.
Le mélange violet est agité 15 minutes à 0°C puis hydrolysé par 100 ml d'eau.
La phase organique inférieure est concentrée pour obtenir 16,5 g d'une huile marron qui est utilisée sans purification.
Ce composé (IX) brut est dissous dans 140 ml d'acide acétique et placé dans un autoclave de 250 ml avec 1,5 g de catalyseur Palladium à 10 % sur charbon et 80 mg d'acide sulfurique concentré.
Le mélange est chauffé à 90°C sous pression de 6 bars d'hydrogène et agité pendant 3 heures.
Après refroidissement, le mélange est filtré puis concentré sous pression réduite.
Le résidu est cristallisé dans 30 ml de diisopropylether, pour obtenir 8,3 g de solide gris.
F = 140°C
Rendement = 68 % à partir de Q₀.

### Exemple 2

### Obtention de l'amide (Ia) où R' = nC₈H₁₇

On prépare une solution de lactone (VI), obtenue selon l'exemple 1 :
2 g (8,03 mmole) dans 20 ml de pyridine, on ajoute 1,0 g (7,8 mmole) de n-octylamine et 2,14 g (10,4 mmole) de dithionite de sodium.
Le mélange hétérogène vert est chauffé à 90°C et agité 1 heure sous atmosphère d'azote puis refroidi, hydrolysé par 50 ml d'eau, extrait par 100 ml de chloroforme.
La phase organique est lavée par 60 ml d'acide chlorhydrique aqueux 5N puis concentrée sous vide, pour conduire à un résidu qui est cristallisé dans 20 ml de diisopropylether, pour obtenir 1,17 g de solide blanc.
F = 92-93°C.
Rendement = 36 % à partir de la n-octylamine.

De la même manière la n-decylamine et la n-dodecylamine ont été condensées avec la lactone de formule (VI), pour obtenir les résulats suivants :

| Amine | F (°C) | Rdt isolé |
|---|---|---|
| R' = nC₁₀H₂₁ | 99-100 | 52 % |
| R' = nC₁₂H₂₅ | 114 | 61% |

### Exemple 3

### Obtention de l'amide (Ib) où R" = nC₁₀H₂₁

Le mode opératoire de l'exemple 2 utilisant comme amine l'amino-2 dodécanol-1 a conduit à l'amide (Ib), avec R" = C₁₀H₂₁.
F = 141-142 °C
Rendement = 55 % à partir de l'amino-2 dodécanol-1.

### Exemple 4

### Obtention de l'amide (Ic) où R'''= H

Le mode opératoire de l'exemple 2 utilisant comme amine l'amino-2 propanediol-1,3 a conduit à l'amide (Ic), R''' = H, isolé après chromatographie sur gel de silice au moyen d'un mélange dichlorométhane-méthanol 95/5 sous forme d'une huile.
Titre RMN ¹H = 93 %
Rendement = 17 % à partir de l'amino-2 propanediol-1,3.

L'effet inhibiteur des radicaux libres revendiqué pour les composés (Ia) et (Ib) obtenus à l'état pur, a été évalué par mesure de leurs absorbances en spectrophotométrie UV à 516 nanomètres en présence de radical diphénylpicrylhydrazide (XI).

On a préparé des solutions éthanoliques contenant 200 micromoles par litre de composé à tester et 100 micromoles par litre de radical (XI) agitées 30 minutes à 20°C et placées dans une cuve de 1 cm de longueur et on a mesuré les absorbances.
a) Mise en évidence de l'effet inhibiteur par rapport à la référence :

| | Gallate de méthyle | Amide (Ib) | (Ia) R'= C₁₂H₂₅ | (Ia) R'= C₁₀H₂₁ | (Ia) R'= C₈H₁₇ |
|---|---|---|---|---|---|
| Absorbance à 516 nm | 0,06 | 0,06 | 0,06 | 0,06 | 0,06 |

b) Dosage de l'activité antiradicalaire des amides (Ia) et (Ib) :

Dans les conditions de mesure d'absorbance à 516 nanomètres, on place dans la cellule UV 2 micromoles de référence ou de substrat (Ia) et (Ib).
On introduit une solution de 200 micromoles de radical (XI), jusqu'à obtention de la coloration violette caractéristique du radical et on mesure l'absorbance du milieu :

| | Gallate de méthyle | Amide (Ib) | (Ia) R'= C₁₂H₂₅ | (Ia) R'= C₁₀H₂₁ | (Ia) R'= C₈₀H₁₇ |
|---|---|---|---|---|---|
| (XI) consommé | 27,7 ml | 14,1 ml | 14,7 ml | 14,3 ml | 14,5 ml |
| Absorbance à 516 nm | 0,34 | 0,52 | 0,34 | 0,39 | 0,45 |

Les composés nouveaux (Ia) et (Ib) présentent une activité anti-radicaux libres voisine qui correspond en équivalent molaire à la moitié de celle du gallate de méthyle pris comme référence.

### Exemple 5

### Oxydation des hydroquinone-amides (I) en quinones (X) où R' = nC₈H₁₇

On prépare une solution du composé (Ia) où R = nC₈H₁₇ selon l'exemple 2 :
60 mg (0,17 mmole) dans 4,5 ml d'éthanol et 0,5 ml d'eau.
On ajoute 27,5 mg (0,17 mmole) de chlorure ferrique.
Le mélange est agité sous courant d'air pendant 2 heures à 20°C.
Le milieu est concentré sous vide, redissout dans 20 ml d'acétate d'éthyle et lavé par 10 ml d'une solution aqueuse 1N d'acide chlorhydrique.
La phase organique est concentrée pour obtenir 57 mg de solide jaune.
F = 103-104°C.
Rendement = 95 % à partir de l'hydroquinone-amide.

La même technique d'oxydation appliquée aux composés hydroquinone-amides (I) dans les exemples 2, 3 et 4, a conduit aux résultats suivants :

| | | F (°C) | Rdt |
|---|---|---|---|
| Amide (Ia) | R'=C₁₀H₂₁ | 100-101 | 98 |
| | R' = C₁₂H₂₅ | 108-109 | 97 |
| Amide (Ib) | R'' = C₁₀H₂₁ | 134-135 | 95 |
| Amide (Ic) | R'''=H | Huile | 98 |

## Revendications

1. Composés hydroquinone-amides dérivés d'un acide arylacétique comportant au moins deux groupes hydroxy et d'amines ou aminoalcanols correspondant à la formule (I) 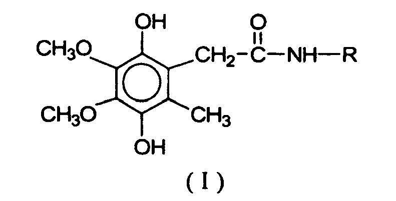 avec R = alkyl (-CₙH₂ₙ₊₁)
ou R = hydroxyalkyl (-CₙH₂ₙ₊₁O)
ou R = dihydroxyalkyl (-CₙH₂ₙ₊₁O₂)
n étant compris entre 1 et 30.

2. Procédé de préparation des composés hydroquinone-amides de formule (I) selon la revendication 1), **caractérisé en ce que** l'on fait réagir l'acide dihydroxy-2,5 diméthoxy-3,4 méthyl-6 benzèneacétique sous forme de lactone (VI) avec une amine, un aminoalcanol ou un aminoalcanediol.

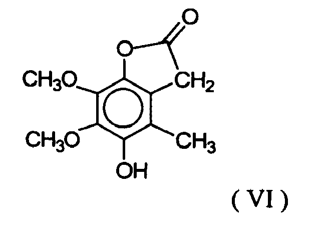

3. Procédé selon la revendication 2) **caractérisé par le fait que** le groupe R de la formule (I) est constitué d'une chaîne alkyle R' linéaire ou branchée contenant de 1 à 30 atomes de carbone, conduisant aux composés (Ia)

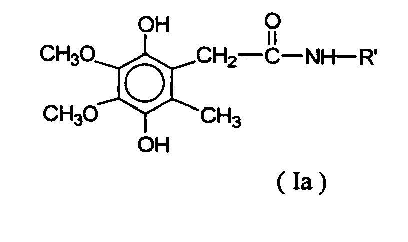

4. Procédé selon la revendication 2) **caractérisé par le fait que** le groupe R de la formule (I) est constitué d'une chaîne hydroxyalkyle où le substituant R" est un atome d'hydrogène ou une chaîne alkyle linéaire ou branchée de 1 à 28 atomes de carbone, conduisant aux composés (Ib)

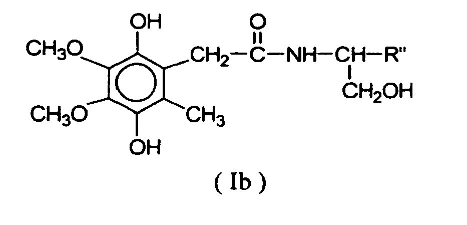

5. Procédé selon la revendication 2) **caractérisé par le fait que** le groupe R de la formule (I) est constitué d'une chaîne dihydroxyalkyle où le substituant R''' est un atome d'hydrogène ou une chaîne alkyle linéaire ou branchée de 1 à 27 atomes de carbone, conduisant aux composés (Ic)

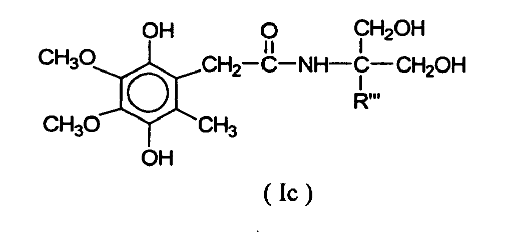

6. Procédé d'obtention et de purification des composés hydroquinone-amides de formule (I) selon la revendication 2) **caractérisé par le fait que** les composés amine RNH₂ sont condensés avec le dérivé lactone (VI) par chauffage dans un solvant polaire aprotique en présence d'un agent réducteur, suivi d'hydrolyse et de cristallisation ou chromatographie.

7. Procédé selon les revendications 2) et 6) où la température du milieu de condensation est comprise entre 50°C et 150°C.

8. Procédé selon les revendications 2), 6) et 7) où la condensation est effectuée en présence d'un agent réducteur du groupe des sulfites alcalins.

9. Utilisation des dérivés hydroquinone-amides de formule (I) tels que définis selon la revendication 1), pour la fabrication de préparations cosmétiques ou pharmaceutiques destinées à la prévention des dégradations biologiques dues aux radicaux libres.

## Claims

1. Hydroquinone-amide compounds derived from an arylacetic acid comprising at least 2 hydroxy and amine or amino-alcohol groups corresponding to formula (I) 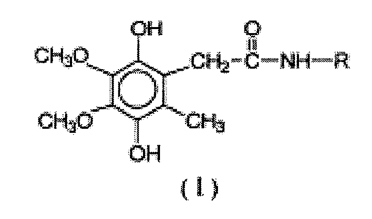 with R = alkyl (-CₙH₂ₙ₊₁)
or R = hydroxyalkyl (-CₙHₙ₂₊₁O)
or R = dihydroxyalkyl (-CₙHₙ₂₊₁O₂)
n being a number between 1 and 30.

2. Process for preparing hydroquinone-amide compounds of formula (I) as in claim 1 in which dihydroxy-2,5 dimethoxy-3,4 methyl-6 benzene acetic acid in the form of lactone (VI) is reacted with an amine, an amino-alcohol or an amino-alkanediol.

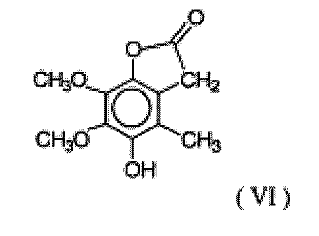

3. Process according to claim 2 in which the R group of formula (I) is composed of a linear or branched alkyl chain R' containing from 1 to 30 carbon atoms, leading to compounds (Ia)

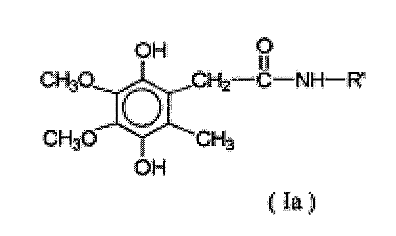

4. Process according to claim 2 in which the R group of formula (I) is composed of a hydroxyalkyl chain in which the R" substitute is a hydrogen atom or a linear or branched alkyl chain of 1 to 28 carbon atoms, leading to compounds (Ib).

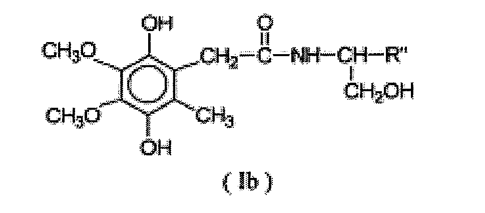

5. Process according to claim 2 in which the R group of formula (I) is composed of a dihydroxyalkyl chain in which the R''' substitute is a hydrogen atom or a linear or branched alkyl chain of 1 to 27 carbon atoms, leading to compounds (Ic).

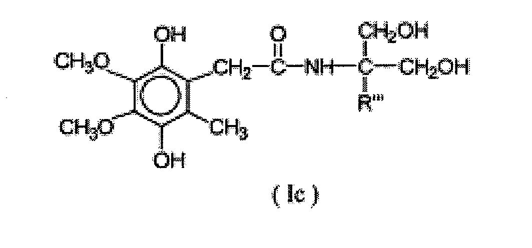

6. Process for obtaining and purifying formula (I) hydroquinone-amide compounds according to claim 2 in which the RNH₂ amine compounds are condensed with the lactone derivative (VI) by heating in an aprotic polar solvent in the presence of a reducing agent, followed by hydrolysis and crystallization or chromatography.

7. Process according to claim 2 in which the temperature of the condensation medium is between 50°C and 150°C.

8. Process according to claims 2, 6 and 7 in which condensation is performed in the presence of a reducing agent from the alkaline sulfite group.

9. Use of formula (I) hydroquinone-amide derivatives as defined according to claim 1 for the manufacture of cosmetic or pharmaceutical preparations intended to prevent the biological degradations caused by free radicals.

## Patentansprüche

1. Aus einer Arylessigsäure abgeleitete Hydrochinonamidverbindungen, die mindestens zwei Hydroxy- und Amin- oder Aminoalcanolgruppen aufweisen, die der Formel (I) entsprechen 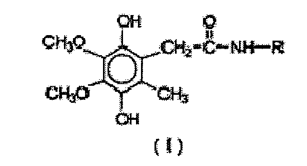 mit R = Alkyl (-CₙH₂ₙ₊₁)
oder R = Hydroxyalkyl (-CₙH₂ₙ₊₁O)
oder R = Dihydroxyalkyl (-CₙH₂ₙ₊₁O₂),
wobei n zwischen 1 und 30 inklusive ist.

2. Verfahren zur Zubereitung von Hydrochinonamidverbindungen der Formel (I) nach Anspruch 1, **dadurch gekennzeichnet, dass** man die Dihydroxy-2,5 dimethoxy-3,4 methyl-6 benzolessigsäure in Form von Lacton (VI) mit einem Amin, einem Aminoalcanol oder einem Aminoalcanediol reagieren lässt.

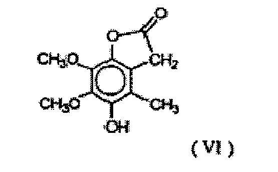

3. Verfahren nach Anspruch 2, **dadurch gekennzeichnet, dass** die R-Gruppe der Formel (I) von einer linearen oder verzweigten R'-Alkylkette gebildet wird, die 1 bis 30 Kohlenstoffatome enthält, die zu Verbindungen (la) führt.

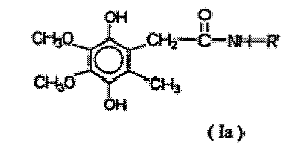

4. Verfahren nach Anspruch 2, **dadurch gekennzeichnet, dass** die R-Gruppe der Formel (I) von einer Hydroxyalkylkette gebildet wird, wobei der Substituent R" ein Wasserstoffatom ist oder eine lineare oder verzweigte Alkylkette mit 1 bis 28 Kohlenstoffatomen, die zu Verbindungen (Ib) führt

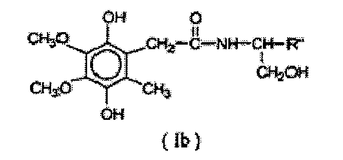

5. Verfahren nach Anspruch 2, **dadurch gekennzeichnet, dass** die R-Gruppe der Formel (I) aus einer Dihydroxyalkylkette besteht, wobei der Substituent R''' ein Wasserstoffatom oder eine lineare oder verzweigte Alkylkette mit 1 bis 27 Kohlenstoffatomen ist, die zu Verbindungen (Ic) führt

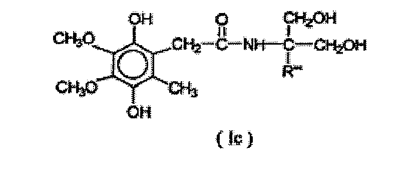

6. Verfahren zur Herstellung und Reinigung von Hydrochinonamidverbindungen der Formel (I) nach Anspruch 2, **dadurch gekennzeichnet, dass** die Aminverbindungen RNH₂ mit dem Lactonderivat (VI) durch Erhitzen in einem polaren aprotischen Lösungsmittel in Anwesenheit eines Reduktionsmittels kondensiert werden, gefolgt von Hydrolyse und von Kristallisierung oder Chromatographie.

7. Verfahren nach den Ansprüchen 2 und 6, wobei die Temperatur des Kondensationsmediums zwischen 50 °C und 150 °C inklusive ist.

8. Verfahren nach den Ansprüchen 2, 6 und 7, wobei die Kondensation in Anwesenheit eines Reduktionsmittels der Gruppe der alkalischen Sulfite durchgeführt wird.

9. Verwendung der Hydrochinonamidderivate der Formel (I), so wie nach Anspruch 1 definiert, zur Herstellung von kosmetischen oder pharmazeutischen Zubereitungen zur Vorbeugung gegen biologische Schäden durch freie Radikale.
